# EUROPÄISCHE PATENTSCHRIFT

(11) **EP 1 940 482 B1**
(45) Veröffentlichungstag und Bekanntmachung des Hinweises auf die Patenterteilung: **23.07.2014**
(21) Anmeldenummer: 06804811.5
(22) Anmeldetag: 17.10.2006
(51) Int. Cl.: A61L 27/32, A61L 27/54

(54) **IMPLANTAT ENTHALTEND AMINO-BISPHOSPHONATE UND VERFAHREN ZU DESSEN HERSTELLUNG**
IMPLANT COMPRISING AMINO-BISPHOSPONATES AND PRODUCTION METHOD FOR SAID IMPLANT
IMPLANT COMPRENANT DES AMINO BISPHOSPHONATES ET PROCEDE DE FABRICATION

(30) Priorität: 27.10.2005 CH 17242005
(43) Veröffentlichungstag der Anmeldung: 09.07.2008
(73) Patentinhaber: Nexilis AG, 2540 Grenchen (CH)
(72) Erfinder: SCHLOTTIG, Falko, CH-4414 Füllinsdorf (CH); SCHNABELRAUCH, Matthias, 07749 Jena (DE); KAUTZ, Armin, Rex, 07745 Jena (DE)
(74) Vertreter: Bremi, Tobias Hans
(86) Internationale Anmeldenummer: PCT/CH2006/000576
(87) Internationale Veröffentlichungsnummer: WO 2007/048263

(56) Entgegenhaltungen:
- WO-A-02/03963
- WO-A-02/098307
- WO-A-2005/018699
- US-A- 4 980 171

## Beschreibung

### TECHNISCHES GEBIET

Die vorliegende Erfindung betrifft ein Implantat, welches wenigstens bereichsweise in im implantierten Zustand mit Hart- und/oder Weichgewebe in Kontakt befindlichen Oberflächenbereichen eine Beschichtung aufweist.

### STAND DER TECHNIK

Verletzte oder beschädigte Teile des Hart- und/oder Weichgewebes des menschlichen Körpers werden am Besten wiederhergestellt oder mechanisch verstärkt, indem körpereigenes Hart- und/oder Weichgewebe verwendet wird. Dies ist aus verschiedenen Gründen nicht immer möglich, und daher kommt in vielen Fällen synthetisches Material als temporäres (bioabbaubares respektive postoperativ entfernbares) oder permanentes Ersatzmaterial zum Einsatz.

Implantate, welche im Hart- und/oder Weichgewebe verankert werden, dienen dem temporären oder permanenten Ersatz oder dem Support von unfall-, abnützungs-, mangelerscheinungs- oder krankheitsgeschädigten oder sonst degenerierten Teilen des menschlichen Körpers. Als Implantat wird normalerweise ein künstliches, chemisch stabiles Material bezeichnet, welches als plastischer Ersatz oder zur mechanischen Verstärkung in den Körper eingebracht wird (vgl. z.B. Roche Lexikon Medizin, Urban & Fischer, (Hrsg.); 5. Aufl. 2003). Die Hilfs- und Ersatzfunktion im Körper wird auf Basis der mechanischen Eigenschaften und des Implantatdesigns übernommen. So sind beispielsweise Hüft- und Kniegelenksprothesen, Wirbelsäulenimplantate und Gefässprothesen seit vielen Jahren im erfolgreichen klinischen Einsatz. Für die Implantatverankerung und die Implantatverträglichkeit an der Grenzfläche Implantatoberfläche / angrenzendes Gewebe hat die Implantatoberfläche eine grosse Bedeutung. Durch eine Veränderung der Implantatoberfläche kann der Heilungsprozess beschleunigt werden.

Zur Oberflächenbehandlung und Oberflächenstrukturierung werden verschiedenste Methoden verwendet, siehe z.B. Titanium in Medicine, Material Science, Surface Science, Engineering, Biological Responses and Medical Applications Series: Engineering Materials, (Brunette, D.M.; Tengvall, P.; Textor, M.; Thomsen, P.(Eds.)); und die darin genannten Referenzen.

Gut etabliert ist beispielsweise die Erhöhung der Rauhigkeit (für viele vgl. Titanium in Medicine, Material Science, Surface Science, Engineering, Biological Responses and Medical Applications Series: Engineering Materials, (Brunette, D.M.; Tengvall, P.; Textor, M.; Thomsen, P. (Eds.)).

Weiterhin gibt es Arbeiten, welche die chemische Modifikation von Implantatoberflächen beschreiben, um eine bessere Anbindung des Knochens an die Implantatoberfläche zu erzielen (Xue W, Liu X, Zheng X, Ding C, Biomaterials. 2005 Jun; 26 (16): 3029-37).

Neuere Ansätze sind pharmazeutische Modifikationen der Oberfläche, um die Osteointegration der Implantate zu beschleunigen und/oder die Regeneration des umliegenden Hart und/oder Weichgewebes zu fördern oder zu stimulieren, beispielsweise mit Wachstumsfaktoren (Raschke MJ, Schmidmaier G. Unfallchirurg. 2004 Aug; 107(8): 653-63).

Im Gegensatz dazu können mit aktiven Substanzen beladene Schichten dazu dienen, unerwünschte Reaktionen auf Implantaten, beispielsweise Gefässprothesen, zu verhindern (Hausleiter J, et al , Eur. Heart J. 2005 Aug; 26(15) : 1475-81. Epub 2005 Jun 23).

Andere für pharmazeutische Oberflächenmodifikation interessante Medikamentgruppen sind Pharmazeutika, die zur systemischen Behandlung von Osteoporose entwickelt wurden, wie beispielsweise Calcitonin, Strontiumranelat und verschiedene Bisphosphonate.

Bisphosphonate können als Strukturanaloga des Pyrophosphats aufgefasst werden, bei denen die P-O-P-Gruppierung durch eine enzymatisch stabile P-C-P-Gruppierung ersetzt ist. Durch Substitution der Wasserstoffatome am C-Atom der P-C-P-Gruppierung sind Bisphosphonate mit unterschiedlichen Strukturelementen und Eigenschaften zugänglich. Bekannte, zur klinischen Anwendung zugelassene Bisphosphonate sind beispielsweise Pamidronsäure, Alendronsäure, Ibandronsäure, Clodronsäure oder Etidronsäure. In der Medizin haben sich Bisphosphonate zur Behandlung von metabolischen Knochenerkrankungen, insbesondere Tumorassoziierten Hypercalcämien, osteolytischen Knochenmetastasen sowie postmenopausalen und glucocorticoinduzierten Osteoporosen etabliert. Weiterhin zeigen Untersuchungen, dass Bisphosphonate zur Prävention oder zur Behandlung von vaskulärer Restenose verwendet werden können (WO00/003677).

In Abhängigkeit von ihrer Struktur unterscheiden sich die bekannten Bisphosphonate zum Teil deutlich in ihrer therapeutischen Wirksamkeit. Eine hohe therapeutische Wirkung besitzen insbesondere solche Bisphosphonate, die in der Struktureinheit zwischen den beiden Phosphoratomen eine Amino-Funktion besitzen. Im folgenden werden diese Verbindungen als Amino-Bisphosphonate bezeichnet.

Die pharmakologische Wirkung der Bisphosphonate beruht auf einer hohen Affinität zu Calciumphosphat-Strukturen der Knochenoberfläche, in deren Folge knochenabbauende Zellen (Osteoklasten) gehemmt werden, was zu einer Verminderung der Knochenresorption und gleichzeitig einer Reaktivierung knochenaufbauender Zellen (Osteoblasten) führt. Auf Grund der speziellen Pharmakokinetik der Bisphosphonate ist eine lokale Therapie der systemischen Gabe vorzuziehen.

Basierend auf diesem Kenntnisstand sind in den letzten Jahren zahlreiche Untersuchungen durchgeführt worden, in denen die Immobilisierung von ausgewählten Bisphosphonaten auf Hartgewebsimplantaten sowie deren Auswirkung auf das Einwachsverhalten des jeweiligen Implantats untersucht wurden.

So wurde beispielsweise in der US 5,733,564 die Beschichtung von Materialien (Endoprothesen, Schrauben, Stifte, etc.) mit wässrigen Bisphosphonat-Lösungen mit dem Ziel beschrieben, die Knochen-Neubildung um das Implantat zu beschleunigen. Die schlechte Haftung der Bisphosphonate auf Metalloberflächen und ihre Wasserlöslichkeit stellen jedoch einen Nachteil dieser Vorgehensweise dar.

Yoshinari et al. (Biomaterials 23 (2002), 2879-2885) zeigten an Hand von in vivo-Studien, dass Galciumphosphat-beschichtete Implantate aus Rein-Titan, die mit einer wässrigen Pamidronat-Lösung imprägniert wurden, eine verbesserte Osteogenese an der Implantatoberfläche aufwiesen, als nicht mit Pamidronat imprägnierte Implantate. Auf Grund der hohen Affinität der Bisphosphonate zu Calciumionen-haltigen Substraten stellen Calciumphosphat-Oberflächen ein mögliches Substrat für die Immobilisierung von Bisphosphonaten dar, da auf diesen Oberflächen die Bioverfügbarkeit der Bisphosphonate und damit ihre therapeutische Wirksamkeit durch Wechselwirkung mit Calcium-Ionen in höherem Masse gegeben ist, als auf weitgehend Calciumionen-freien Oberflächen.

Eine weitere Variante der Immobilisierung von Bisphosphonaten in Hydroxyapatithaltigen Beschichtungen von Knochenimplantaten beschreibt WO-A-02/04038. Da metallische Implantate im Hartgewebebereich eine dominierende Rolle spielen und andererseits eine Calciumphosphat-Beschichtung von Metalloberflächen erhöhte Fertigungsaufwendungen mit sich bringt, wurden in letzter Zeit zahlreiche Versuche unternommen, metallische Implantatmaterialien so zu modifizieren, dass auf ihnen eine wirksame Bisphosphonat-Immobilisierung ermöglicht wird.

So wurden Arbeiten bekannt, in denen Calciumionen mittels Elektronenstrahl-Implantation in die Oberfläche von Titanimplantaten eingebracht werden (JP 2000070288, H. Kajiwara et al. Biomaterials 26 (2005), 581-587), um eine verbesserte Anhaftung von Bisphosphonaten zu erzielen. Dieses Verfahren hat allerdings den Nachteil eines hohen apparativen Aufwandes.

Weitere Arbeiten beschäftigen sich mit der elektrolytischen Abscheidung von Calcium-Etidronat auf Reintitan (K. Duan et al. J. Biomed. Mater. Res.: Appl. Biomater. 72B (2005), 43-51), wobei zwar dünne Filme aus Bisphosphonat abgeschieden werden konnten, die jedoch Inhomogenitäten aufweisen und beim Trocknen Schrumpfungserscheinungen zeigten.

In WO-A-2005/018699 werden Bisphosphonat-beschichtete metallische Implantate beschrieben, die in der Weise hergestellt werden, dass zunächst eine Proteinschicht, beispielsweise aus Fibrinogen, auf die Metalloberfläche immobilisiert wird. An diese Proteinschicht werden anschliessend ein oder mehrere Bisphosphonate kovalent über reaktive funktionelle Gruppen gebunden. Ein wesentlicher Nachteil dieser Methode liegt in der Verwendung toxischer Reagenzien bei der Immobilisierung bzw. Vernetzung der Proteinschicht und der kovalenten Anbindung des Bisphosphonats.

Die WO 02/098307 betrifft Implantate, welche ein Bisphosphonat enthalten oder mit diesem beschichtet sind. Ohne spezifischen Bezug zu einer dieser beiden Alternativen wird u.a. vorgeschlagen, das Bisphosphonat mit anderen Komponenten in Mischung vorzulegen.

### DARSTELLUNG DER ERFINDUNG

Der Erfindung liegt demnach u.a. die Aufgabe zugrunde, ein verbessertes Implantat zur Verfügung zu stellen, welches z.B. eine gute sowie komplikationslose Osteointegration zeigt, und welches dennoch in einem einfachen und kostengünstigen Verfahren hergestellt werden kann.

Eine Lösung dieser Aufgabe wird beispielsweise dadurch erreicht, dass das Implantat wenigstens bereichsweise in im implantierten Zustand mit Hart- und/oder Weichgewebe wenigstens mittelbar in Kontakt befindlichen Oberflächenbereichen eine Beschichtung aufweist. Wenigstens mittelbar in Kontakt bedeutet dabei, dass die Beschichtung direkt mit dem Hart- und/oder Weichgewebe in Kontakt sein kann, oder aber auch über Kanäle, Öffnungen und/oder eine weitere Schicht oder Schichten, welche aber die unten beschriebene Freisetzungscharakterisitk des Bisphosphonates nicht oder nur unwesentlich beeinflussen resp. ändern. Diese Beschichtung enthält sowohl wenigstens ein Amino-Bisphosphonat nach Anspruch 1, als auch wenigstens eine amphiphile und/oder eine wasserlösliche ionische polymere Komponente nach Anspruch 1.

Auch Mischungen von unterschiedlichen derartigen Amino-Bisphosphonaten sind möglich sowie Mischungen von unterschiedlichen amphiphilen Komponenten resp. wasserlöslichen ionischen polymeren Komponenten.

Als Substituenten für die Alkylgruppe von Y kommen auch kationische C2-C5 Ammoniumderivate in Frage wie z.B. N(CH₂CH₃)₃.

Y ist eine lineare C1 - C7 Alkylgruppe substituiert durch NH₂, N(CH₃)₂, NH(CH₃), N(CH₃)₃, Pyridinyl oder Imidazolyl. Die amphiphile Komponente ist ein lineares unsubstituiertes C10-C20 Alkyl-Carboxylat oder Alkyl-Sulfat.

Einer der Kerne der Erfindung besteht somit darin, das Bisphosphonat, welches ohne spezifische Massnahmen infolge der grossen Löslichkeit in wässrigen Lösungen zu mobil ist und nach dem Einbau des Implantates zu schnell von der Oberfläche weggetragen würde, in einem Mischsalz mit einer zweiten Komponente zu mischen resp. zu binden, was dazu führt, dass dieses Mischsalz, welches in Wasser, und damit auch in der physiologischen Umgebung nach dem Einbau des Implantates, eine wesentlich niedrigere Löslichkeit aufweist, somit die Wirksamkeit über eine wesentlich längere Zeitdauer an der entscheidenden Grenzfläche ausüben kann. Es wird festgestellt, dass bei Verwendung der Beschichtung nach der Erfindung überraschenderweise die Verfügbarkeit des in der Beschichtung enthaltenden Bisphosphonates an der Implantatoberfläche beziehungsweise in der direkten Umgebung des Implantates während mehrerer Tage bis Wochen gewährleistet ist. Überraschenderweise lässt sich dies durch eine spezifische Auswahl von Zusatzkomponenten erreichen. Die amphiphile Komponente respektive das Bisphosphonat und die wasserlösliche ionische polymere Komponente liegen als Mischsalz (d.h. die amphiphile Komponente ist ebenfalls ionisch) mit geringer Löslichkeit in Wasser, vor, und es zeigt sich, dass durch die Verwendung der spezifischen amphiphilen respektive wasserlöslichen ionischen polymeren Komponente eine erstaunlich gute Haftung des Bisphosphonates auf gängigen Implantat-Materialien erreichbar ist. Es handelt sich bevorzugtermassen bei der Beschichtung um eine trockene Beschichtung.

Die vorliegende Erfindung beruht somit u.a. auf der Überlegung, dass die Freisetzung eines niedermolekularen Wirkstoffs aus einer Implantatbeschichtung in das umgebende, im Falle eines Implantates wässrige Milieu, wesentlich durch seine Diffusion aus der trockenen Schicht in die Umgebung bestimmt wird, und dass diese Freisetzung wiederum von der Löslichkeit des Wirkstoffs im umgebenden wässrigen Medium bestimmt wird. Bisphosphonate, sind in der Regel gut wasserlösliche Verbindungen, so dass mit einer schnellen Diffusion aus einer Anfeuchtung und gleichermassen aus einer trockenen Beschichtung und damit mit einer geringen Retardierung des Wirkstoffs am Wirkort zu rechnen ist. Es ist deshalb einer der Kerngedanken der vorliegenden Erfindung, den ursprünglich bereits schon als Lösung oder in einer leicht löslichen Salzform vorliegenden und auf diese Weise entsprechend dem Stand der Technik eingesetzten Wirkstoff in eine schwerlösliche Salzform in einer trockenen Schicht zu überführen. Die Verfügbarkeit des Wirkstoffs wird dann durch ein Löslichkeitsgleichgewicht zwischen ursprünglichem freien Wirkstoff und dem in Form eines unlöslichen Salzes vorliegenden Wirkstoff bestimmt. Wenn nun im wässrigen Medium der entsprechend des Löslichkeitsprodukts des schwerlöslichen Wirkstoffsalzes frei verfügbare Wirkstoff aus der Beschichtung heraus diffundiert, verschiebt sich das Gleichgewicht zu Gunsten des freien Wirkstoffs und es erfolgt auf diese Weise eine allmähliche Freisetzung des Wirkstoffs aus dem schwerlöslichem Wirkstoffsalz. Es ist mit anderen Worten, dem Diffusionsgleichgewicht ein Löslichkeitsgleichgewicht vorgelagert und das Herauslösen des Wirkstoffs aus dem schwerlöslichem Wirkstoffsalz löst die Diffusion als geschwindigkeitsbestimmenden Schritt der Wirkstofffreisetzung ab. Voraussetzung für die Anwendung dieses Konzeptes ist die Fähigkeit der Bisphosphonate, in wässrigem Medium schwerlösliche Salze mit entsprechenden anionischen oder kationischen Reaktionspartnern, der erfindungsgemäss vorgeschlagenen amphiphilen ionischen respektive wasserlöslichen ionischen polymeren Komponente zu bilden.

In den genannten Salzen aus Amino-Bisphosphonaten und der amphiphilen ionischen, d.h. anionischen Komponente, spezifisch den langkettigen Alkan-sulfaten resp. - carboxylaten bildet das jeweilige Bisphosphonat die kationische Komponente und die amphiphile ionische respektive wasserlösliche ionische polymere Komponente, spezifisch das jeweilige langkettige Carboxylat bzw. Alkansulfat die anionische Komponente. Es wurde weiterhin gefunden, dass durch den gleichzeitigen oder nachträglichen Zusatz eines wasserlöslichen Salzes wie z.B. eines Calcium- und/oder Strontiumsalzes die Wasserlöslichkeit des betreffenden Salzes aus Amino-Bisphosphonaten und langkettigen Carbonsäuresalzen oder langkettigen Alkan-sulfaten weiter verringert werden kann. Erfindungsgemäss ist auch die Verwendung langkettiger Carbonsäuren sowie langkettiger Alkyl-Schwefelsäuren anstelle der entsprechenden wasserlöslichen Salzformen.

Der Erfindung liegt, wie bereits weiter oben erwähnt, u.a. weiterhin der überraschende Befund zugrunde, dass Amino-Bisphosphonate mit den beanspruchten wasserlöslichen ionischen Polymeren, die sich von an sich bekannten biologisch verträglichen (Bio-)Polymeren ableiten, in Wasser gering lösliche Bisphosphonat-Polymer-Salze bilden, die ebenfalls auf nichtmetallischen oder metallischen Oberflächen haften, ohne dass weitere Schichtbildner oder ein Träger (Carrier) erforderlich sind. Die genannten Salze aus Amino-Bisphosphonaten und langkettigen Carbonsäuren oder langkettigen Alkan-sulfaten sowie die genannten Bisphosphonat-Polymer-Salze eignen sich als Beschichtungen für nichtmetallische oder metallische Oberflächen und setzen im wässrigen Medium Bisphosphonat retardiert frei.

Es ist z.B. erfindungsgemäss, dass die genannten Salze aus Amino-Bisphosphonaten und langkettigen Carbonsäuren oder langkettigen Alkan-sulfaten sowie die genannten Bisphosphonat-Polymer-Salze als feinverteilte Suspensionen aus Wasser oder leichtflüchtigen, organischen Lösungsmitteln, wie z. B. aus Chloroform oder Chloroform-Mischungen durch ein Beschichtungsverfahren, also beispielsweise durch Tauchen, Sprühen oder Tropfen auf nichtmetallische oder metallische oder native oder polymere Oberflächen aufgebracht werden können, wobei sie gut haftende Beschichtungen bilden.

Bei der Beschichtung handelt es sich um eine Beschichtung, welche ohne zusätzlichen Träger respektive zusätzlichen Carrier vorliegt. Mit anderen Worten besteht die Beschichtung im wesentlichen oder gar vollständig nur aus den genannten Mischsalzen. Dies vereinfacht die Herstellung derartiger Implantate wesentlich. Es zeigt sich nämlich überraschenderweise, dass die vorgeschlagenen Mischsalze im Gegensatz zu anderen Wirkstoffen direkt als Beschichtung aufgebracht werden können, und ein zusätzlicher spezifischer Träger oder ein Carrier nicht erforderlich ist.

Die Beschichtung kann in einem geeigneten Lösemittel durch Tauchen, Aufsprühen oder Auftropfen auf die zu beschichtende Oberfläche aufgebracht werden und nach Verdampfung oder Verdunstung des Lösemittels bildet sich durch in situ Salzbildung eine in Wasser gering lösliche bisphosphonat-haltige Beschichtung.

Die Beschichtung zeichnet sich also unter anderem bevorzugtermassen dadurch aus, dass sie nach Einsetzen in das menschliche oder tierische Gewebe respektive in den menschlichen oder tierischen Knochen das Bisphosphonat in verzögerter Weise über einen längeren Zeitraum an die unnittelbare Umgebung des Implantates abgibt respektive in der unmittelbaren Umgebung des Implantates Wirksamkeit entfaltet.

Gemäss einer ersten bevorzugten Ausführungsform verfügt die Mischung respektive das Mischsalz über eine Löslichkeit in reinem Wasser von weniger als 1 mg/ml bei Raumtemperatur, insbesondere bevorzugt im Bereich von 0.05 - 0.9 mg/ml bei Raumtemperatur.

Als Implantat im Sinne dieser Erfindung soll jegliche Trägerstruktur verstanden werden, welche insbesondere zur Verwendung in respektive Einbettung in und/oder an menschliches oder tierisches Gewebe respektive Knochen geeignet ist. Dentalimplantate sind dabei ausdrücklich ausgeschlossen. Das Implantat kann eine glatte oder aufgeraute Oberfläche aufweisen. Es kann porös oder unporös sein, letzteres mit offenen, die gesamte Struktur durchdringenden Poren oder aber mit nur oberflächlicher Porosität. Es kann sich um eine unverstärkte oder verstärkte Trägerstruktur handeln, beispielsweise um eine faserverstärkte Trägerstruktur. Derartige Implantate können zu Beispiel ausgewählt sein aus der Gruppe: Gefässprothese, Knochenersatzmaterial, Knochenunterstützungsmaterial, Scaffold, Folie, Stift, Dorn, Gerüst, Nagel, Draht, Schraube, Platte, Rohr, Schlauch, Polymerschaumkomponente mit offener Porenstruktur, Faser, Gewebe, Prothesen, insbesondere Gelenkprothesen, Netze, Käfige (cages), Metallschäume, poröse metallische und keramische Beschichtungen, allogene und xenogene Implantate, kardiovaskuläre Implantate, künstliche Bänder, künstliche Bandscheiben, intracorneale und intraokulare Implantate, cochleare Prothesen, aktive Implantate, Elektroden und/oder Kombinationen davon. Weiterhin kann das Implantat sogar teilweise oder gänzlich bioabbaubar ausgestaltet sein.

Grundsätzlich werden Trägerstrukturen bevorzugt, welche mittelfristig und langfristig im tierischen oder menschlichen Körper vorgesehen sein sollen. Es ist aber auch möglich, die erfindungsgemässe Beschichtung auf Strukturen aufzubringen, welche nur temporär und gegebenenfalls nur zur zeitlich verzögerten Abgabe von Bisphosphonat im Körper vorgesehen sind. Entsprechend kann es sich beim Implantat nach der Erfindung auch um zum Beispiel beschichtete Kugeln, Fasern, Gewebe, Folie oder ähnliches handeln. Insbesondere in diesem Zusammenhang ist es auch möglich, die eigentliche Trägerstruktur bioabbaubar zu gestalten, so dass sie nach der sukzessiven Abgabe des Wirkstoffs nicht postoperativ entfernt werden muss sondern vom Körper selbstständig aufgelöst und abgebaut wird. So können die Beschichtungen nach der Erfindung auf solchem bioabbaubaren Material beispielsweise nach einem generellen Eingriff im Bereich des operierten Knochens deponiert werden, geben dort sukzessive den Wirkstoff ab solange dieser in dieser Zone erforderlich ist, und werden anschliessend vom Körper abgebaut.

Eine weitere bevorzugte Ausführungsform zeichnet sich dadurch aus, dass es sich beim Amino-Bisphosphonat um Pamidronsäure, Alendronsäure, Neridronsäure, Risedronsäure, Zoledronsäure, Olpadronsäure, Ibandronsäure, Minodronsäure oder Cimadronsäure oder eine Mischung und/oder Alkali- oder Erdalkali-Salze davon. Als besonders wirksam erweisen sich die bereits bekannten Komponenten Pamidronsäure und/oder Alendronsäure gegebenenfalls in Form des Alkali- oder Erdalkali-Salzes, so beispielsweise Natrium-Alendronat respektive Natrium-Pamidronat. Generell ist es bevorzugt, wenn das Bisphosphonat in der freien Phosphonsäure-Form, der Natrium-, Kalium-, Ammonium-, Calcium-, Magnesium- und/oder Strontium-Salzform vorliegt.

Es handelt es sich bei der amphiphilen Komponente, welche im Mischsalz mit dem Bisphosphonat zu einer reduzierten Löslichkeit des Bisphosphonates Anlass gibt, um wenigstens eine Komponente ausgewählt aus der Gruppe der linearen unsubstituierten C10-C20 Alkyl-Carboxylate oder Alkyl-Sulfate respektive deren Alkali- oder Erdalkali-Salze, bevorzugt um Laurat, Stearat, Palmitat, Myristat, Oleat, Behenat, Dodecylsulfat, bevorzugt als Alkali- oder Erdalkali-Salze oder Mischungen davon.

Bei der wasserlöslichen ionischen polymeren Komponente, welche im Mischsalz mit dem Bisphosphonat zu einer reduzierten Löslichkeit des Bisphosphonates Anlass gibt, handelt es sich erfindungsgemäss um eine polymere Komponente mit freien anionischen Gruppen, welche sich von biologisch verträglichen Biopolymeren ableitet. Namentlich um carboxylierte, carboxymethylierte, sulfatierte oder phosphorylierte Derivate natürlicher Polysaccharide, insbesondere bevorzugt von Polysacchariden ausgewählt aus Dextran, Pullulan, Chitosan, Stärke oder Cellulose oder Mischungen davon.

Vorzugsweise liegt das insbesondere bevorzugt als Amino-Bisphosphonat ausgewählte Bisphosphonat und die insbesondere bevorzugt als Alkylsulfat oder Alkylcarboxylat ausgewählte amphiphile Komponente in der Beschichtung in einem Molverhältnis zwischen 10 : 1 und 1 : 5 vor, insbesondere bevorzugt in einem Molverhältnis von 2:1 bis 1:2. Entsprechend liegt das als Amino-Bisphosphonat ausgewählte Bisphosphonat und die wasserlösliche ionische polymere Komponente in der Beschichtung bevorzugtermassen in einem Molverhältnis zwischen 10 : 1 und 1 : 5, insbesondere bevorzugt in einem Molverhältnis von 2:1 bis 1:2, jeweils bezogen auf die Aminogruppen des eingesetzten Aminogruppen-haltigen Bisphosphonats und die vorhandenen anionischen Gruppen der polymeren Komponente, vor.

Eine solche Beschichtung kann auf einer glatten, porösen und/oder aufgerauten Oberfläche aufgebracht sein. Die Oberflächenstruktur kann dabei über mechanische Verfahren (z.B. Sandstahlen) und/oder über chemische Verfahren (z.B. Säurebehandlung) und/oder über chemisch physikalische Verfahren wie z.B. Plasmaspritzen hergestellt sein.

Grundsätzlich lässt sich diese Beschichtung auf Implantate nach dem Stand der Technik auftragen, so beispielsweise auf ein Implantat auf metallischer und/oder keramischer Basis. Es zeigt sich dabei, dass die Beschichtung nicht auf eine spezifische darunter liegende Schicht oder einen zusätzlichen Träger/Carrier angewiesen ist, um das Bisphosphonat zu immobilisieren, was die Herstellung wesentlich vereinfacht und kostengünstiger macht. Entsprechend kann die Beschichtung unmittelbar und ohne Zwischenschicht auf ein derartiges Implantat aufgebracht sein. Beim Implantat handelt es sich zum Beispiel um ein Implantat auf Basis von Calciumphosphat-Keramiken, Bioglass, Glaskeramiken, Calciumcarbonat, Calciumsulfat, organischen und bevorzugt resorbierbaren Polymeren oder Kompositen der genannten Materialien, oder auf Basis von Reintitan, Titanlegierungen, Kobalt-Chrom-Legierungen oder Edelstahl, oder auf Basis von nativen Elementen wie Kollagen, Gelatine oder Materialien allogener Herkunft.

Vorzugsweise verfügt die Beschichtung über eine Dicke im Bereich von 0.1-10 µm, vorzugsweise von 0.5 - 5 µm.

Des weiteren betrifft die vorliegende Erfindung ein Verfahren zur Herstellung eines Implantates, insbesondere von der Art, wie es oben beschrieben wurde. Dabei wird eine Suspension oder Lösung, welche sowohl ein Bisphosphonat der allgemeinen Formel, wie oben angegeben, als auch wenigstens eine amphiphile Komponente, wie oben angegeben, und/oder eine wasserlösliche ionische polymere Komponente, wie oben angegeben, enthält, hergestellt, und die Beschichtung wird durch Tauchen, Aufsprühen oder Auftropfen dieser Suspension oder Lösung (resp. des Suspensions- oder Lösemittelgemisches) auf die zu beschichtende Oberfläche des Implantates aufgebracht und nach Verdampfung oder Verdunstung des Suspensions- oder Lösemittels (bzw. Suspensions- oder Lösemittelgemischs) eine in Wasser gering lösliche Beschichtung gebildet.

Die Beschichtung kann dabei entweder dadurch hergestellt werden, dass in einem ersten Beschichtungsschritt eine Lösung eines Amino-Bisphosphonats in einem geeigneten Lösemittel durch Tauchen, Aufsprühen oder Auftropfen auf die zu beschichtende Oberfläche aufgebracht wird und nach Verdampfung oder Verdunstung des Lösemittels in einem zweiten Beschichtungsschritt eine amphiphile und/oder polymere Komponente in einem geeigneten Lösemittel durch Tauchen, Aufsprühen oder Auftropfen auf die zu beschichtende Oberfläche aufgebracht wird und nach Verdampfung oder Verdunstung des zweiten Lösemittels durch in situ Salzbildung eine in Wasser gering lösliche bisphosphonat-haltige Beschichtung gebildet wird.

Es ist aber auch möglich, die beiden Komponenten zunächst in einer wässrigen Lösung herzustellen, aus dieser auszufällen, und anschliessend gemeinsam mit einem geeigneten Lösemittel oder Suspensionsmittel mit den genannten Methoden aufzubringen. So kann beispielsweise das Bisphosphonat und die amphiphile Komponente und/oder die wasserlösliche ionische polymere Komponente hergestellt werden, indem in Wasser gelöstes Bisphosphonat mit in Wasser gelöster amphiphiler Komponente respektive wasserlöslicher ionischer polymerer Komponente vermischt werden und, gegebenenfalls nach Zugabe von weiteren Salzen, wie beispielsweise Kalziumchlorid, das Fällungsprodukt als Mischsalz isoliert wird, und anschliessend dieses Mischsalz in einem Suspensions- oder Lösemittel (z.B. org. Lösungsmittel wie Chloroform oder auch Wasser) oder Suspensions- oder Lösemittelgemisch aufgelöst respektive in diesem suspendiert werden. Das weitere Salz, welches zur Fällung verwendet wird, kann dabei z. B. in einem Verhältnis Bisphosphonat: weiteres Salz von im Bereich 1:2 bis 2:1 eingesetzt werden.

Die Trocknung der beschichteten Implantate kann durch ein bekanntes Trocknungsverfahren, also beispielsweise durch Trocknung im Gasstrom oder unter Anwendung von Unterdruck und/oder erhöhter Temperatur erfolgten. Erfindungsgemäss ist, dass das Aufbringen der beiden Lösungen auch in umgekehrter Reihenfolge durchgeführt werden kann. Bevorzugtermassen ist es zudem möglich, das Mischsalz auf ein aufgewärmtes Implantat, z.B. bei einer Temperatur des Implantats von mehr als 70 Grad Celsius, aufzubringen.

Erfindungsgemäss lassen sich mit den beschriebenen bisphosphonat-haltigen Zusammensetzungen nichtmetallische und metallische und native Implantatoberflächen beschichten. Im ersten Fall sind Materialien aus Aluminiumoxid- Zirkonoxid, Calciumphosphatkeramiken, Bioglass oder Glaskeramiken oder Mischungen dieser Keramiken und bevorzugt resorbierbaren Polymere, besonders bevorzugt. Im zweiten Fall bestehen sie aus, in der Implantatmedizin üblichen, Reinmetallen oder Metalllegierungen wie beispielsweise Reintitan, Titanlegierungen, Kobalt-Chrom-Legierungen oder Edelstahl. Die nativen Materialien können beispielsweise auch aus kollagen Scaffolds oder Allograftimplantaten bestehen.

Die Verwendung von Implantaten mit strukturierter Oberfläche ist besonders bevorzugt.

Gemäss einer bevorzugten Ausführungsform des erfindungsgemässen Verfahrens werden die Konzentrationen der Beschichtungslösungen die das Amino-Bisphosphosphonat und die amphiphile und/oder die polymere Komponente enthalten, so gewählt, dass in der durch in situ-Salzbildung entstehenden Beschichtung das (Amino-) Bisphosphonat und die amphiphile Komponente respektive die polymere Komponente in einem Molverhältnis zwischen 10 : 1 und 1 : 5, bevorzugt zwischen 2:1 und 1:2, vorliegen.

Als Suspensions- oder Lösemittel oder Suspensions- oder Lösemittelgemisch können neben Wasser ein oder mehrere organische Suspensions- und/oder Lösungsmittel verwendet werden, so z.B. Chloroform als Suspensionsmittel oder eine Mischung aus Chloroform und Triethylenglykol im Verhältnis von 97.5 : 2.5 als Lösungsmittel.

Weitere bevorzugte Ausführungsformen der Erfindung ergeben sich aus den abhängigen Patentansprüchen.

### KURZE ERLÄUTERUNG DER FIGUR

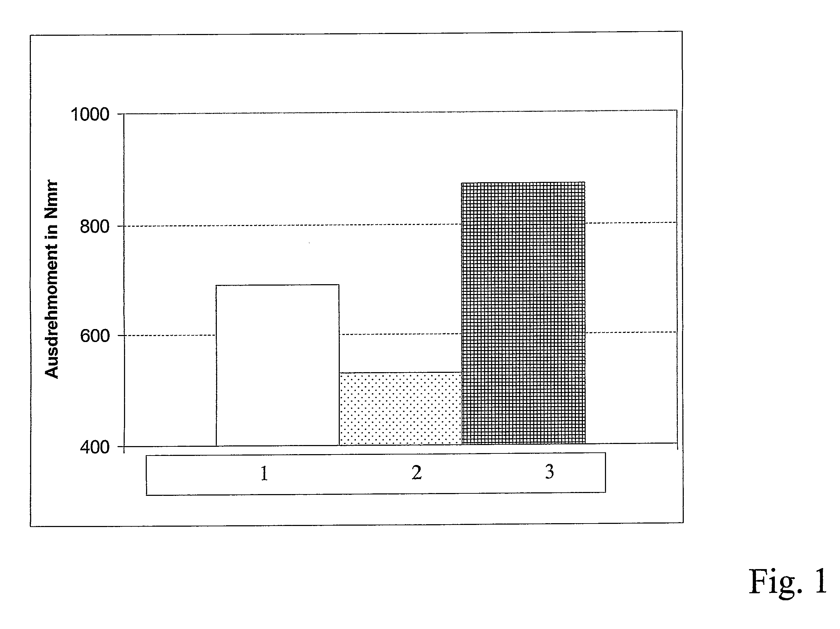

Die Erfindung soll nachfolgend anhand von Ausführungsbeispielen im Zusammenhang mit der Figur näher erläutert werden. Fig. 1 zeigt das Ausdrehmoment für Implantate mit unterschiedlichen Oberflächen.

### WEGE ZUR AUSFÜHRUNG DER ERFINDUNG

Die nachstehenden Beispiele dienen der näheren Erläuterung der Erfindung, ohne sie einzuschränken. Variationen der vorgestellten Ausführungsbeispiele, wie sie durch die nachfolgenden Patentansprüche umfasst werden, sind dem Fachmann im Rahmen seiner technischen Fachkenntnisse zugänglich, und entsprechend sollen die anfolgend gezeigten Ausführungsbeispiele nicht zur Einschränkung des durch die Patentansprüche verliehenen Schutzbereichs sondern nur zur Stützung ausgelegt werden.

### Herstellung eines Alendronsäure-Stearat-Salzes

100 mg (0,3076 mmol) Natrium-Alendronat werden in 10 ml Wasser bei 80°C gelöst und zu einer Lösung von 94,3 mg (0,3076 mmol) Natriumstearat in 5 ml Wasser (gelöst bei 80°C) gegeben. Die sich dabei bildende milchige Suspension wird über 18 Stunden bei 80°C unter inerten Bedingungen gerührt. Die Suspension wird anschliessend 10 min bei 14000 U/min zentrifugiert. Nach dem Abnehmen des Überstandes wird der Niederschlag mit destilliertem Wasser gewaschen und im Exsikkator unter Vakuum (10 mbar) bei Raumtemperatur mindestens 2 Tage getrocknet. Das Endprodukt wurde in einer Ausbeute von 30% erhalten.

### Herstellung eines Calcium-Pamidronat-Stearats

Es werden 20 mg (0,0717 mmol) Dinatriumpamidronat in 5 ml Wasser und 21,97 mg (0,0717 mmol) Natriumstearat in 5 ml Wasser bei jeweils 80°C gelöst. Die beiden klaren Lösungen werden gemischt und 30 min bei 80°C gerührt. Nach Zugabe von 1M Calciumchloridlösung (Verhältnis Pamidronat : Stearat : CaCl₂ = 1 : 1 : 1) bildet sich eine milchig weisse Suspension, die 18 Stunden bei 80°C unter inerten Bedingungen gerührt wird. Anschliessend wird der Niederschlag zentrifugiert (14000 U/min; 10 min) und der Überstand entfernt. Der verbleibende Niederschlag wird einmal mit destilliertem Wasser gewaschen. Das Endprodukt wird im Exsikkator unter Vakuum (10 mbar) mind. 2 Tage getrocknet. Das Calcium-Pamidronat-Stearat wird in einer Ausbeute von 69,3% erhalten.

### Herstellung von Alendronsäure-Dodecylsulfat

100 mg (0,3076 mmol) Natrium-Alendronat werden in 10 ml Wasser bei Raumtemperatur gelöst und zu einer Lösung von 88,7 mg (0,3076 mmol) Natriumdodecylsulfat (SDS) in 5 ml Wasser (gelöst bei RT) gegeben und 30 min bei Raumtemperatur gerührt. Nach Zugabe von 1M Calciumchlorid-Lösung im Verhältnis Alendronat : SDS : CaCl₂ = 1 : 1 : 1 fällt ein weisser Niederschlag aus. Die Suspension wird weitere 18 Stunden bei Raumtemperatur gerührt. Nach Zentrifugation (14000 U/min; 10 min) wird der klare Überstand entfernt und der Niederschlag mit destilliertem Wasser gewaschen. Das Endprodukt wird im Exsikkator unter Vakuum (10 mbar) bei Raumtemperatur mind. 2 Tage getrocknet. Die erzielte Ausbeute an Alendronsäure-Dodecylsulfat beträgt 88,4%.

### Herstellung eines Calcium-Alendronsäure-Carboxymethyldextran-Salzes

Es werden 50 mg (0,15378 mmol) Natrium-Alendronat (in 4 ml Wasser gelöst) mit 22,98 mg (0,1038 mmol) Carboxymethyldextran (CMD) mit einem Substitutionsgrad von 0,74, gelöst in 1 ml Wasser, gemischt und 30 min bei Raumtemperatur gerührt. Nach Zugabe von 1M Calciumchloridlösung im Verhältnis Alendronat : CMD : CaCl₂ = 2 : 1 : 2 bildete sich ein weisser, milchiger Niederschlag. Die Suspension wurde weitere 18 Stunden bei Raumtemperatur gerührt. Nach Zentrifugation (14000 U/min; 10 min) wird der klare Überstand abgenommen und der verbleibende Niederschlag mit destilliertem Wasser gewaschen. Das Endprodukt wird im Exsikkator unter Vakuum (10 mbar) bei Raumtemperatur mind. 2 Tage getrocknet. Das Verhältnis Alendronat zu CMD wurde von 2 : 1 bis 1 : 2 variiert. Die Ausbeuten der jeweiligen Ansätze betrugen 54,2% für 2 : 1, 44,8% für 1 : 1 und 12,2% für 1 : 2.

### Beschichtung von Implantaten

Ein Implantat (Hüftgelenksimplantat) auf Basis von Titan wurde zunächst in einem Sandstrahlverfahren an der dem Knochen ausgesetzten Partie aufgeraut. Anschliessend wurde eine Suspension des oben hergestellten Alendronsäure-Stearat-Salzes in Chloroform durch Zugabe von 0,025g des Alendronsäure-Stearat-Salzes zu 4,975g Chloroform (3,3 ml) unter Rühren innerhalb von 10 min hergestellt. Durch Behandlung mit einem Ultraschall-Homogenisator (20 Watt Gesamtleistung) wurde eine homogene Suspension erhalten.

Die Implantate wurden auf 80°C erwärmt und mit der beschriebenen Suspension mehrfach mit einer konventionellen Sprühpistole (3x) besprüht. Während des Sprühvorgangs rotierten die in eine geeignete Vorrichtung eingespannten Implantate gleichmässig um ihre Längsachse. Zwischen den Aufsprühzyklen wurden die Implantate bei 80°C solange getrocknet, bis das Lösungsmittel vollständig verdampft war.

### Tierversuche

Die so hergestellten Implantate zeigten ein komplikationsloses Einwachsverhalten und eine im Vergleich zu den Implantaten nach dem Stand der Technik verbesserte Osteointegration. Weiterhin zeigt sich auch eine gute Integration an Weichgewebe. Die Figur 1 zeigt entsprechende Resultate von mit drei verschiedenen Oberflächenimplantattypen durchgeführten Versuchen. Dabei wurde ein Titanimplantat in Form eines Stiftes mit einem Durchmesser von 4.2 mm und einer Länge von 8 mm verwendet. Die Oberflächen wurden bei Implantat (1) sandgestrahlt und säuregeätzt ohne weitere Beschichtung, bei Implantat (2) plasmachemisch anodisch oxidiert ohne weitere Beschichtung und bei Implantat (3) sandgestrahlt und säuregeätzt und mit einer Beschichtung im wesentlichen gemäss dem oben beschriebenen Beispiel zur Beschichtung von Implantaten beschichtet und dann die Implantate (1), (2) und (3) im Tierversuch verglichen. Die sandgestrahlt säuregeätzte und die plasmachemisch anodisch oxidierte Oberfläche entsprechen den Oberflächen von kommerziell verbreiteten und häufig verwendeten Implantaten.

Die Implantate wurden in die Beckenschaufel von Schafen implantiert. Nach einer Einheilzeit von 2 Wochen wurde das Ausdrehmoment in Nmm bestimmt, welches erforderlich war, um die eingewachsenen Implantate vom Knochen zu lösen. Wie Figur 1 zeigt, findet man ein signifikant besseres Einwachsen des erfindungsgemäss beschichteten Implantates (3).

## Patentansprüche

1. Implantat unter Ausschluss eines Dentalimplantats, welches wenigstens bereichsweise in im implantierten Zustand mit Hart- und/oder Weichgewebe wenigstens mittelbar in Kontakt befindlichen Oberflächenbereichen eine Beschichtung aufweist, welche sowohl ein
Amino-Bisphosphonat der allgemeinen Formel
(H₂O₃P)-C(X)(Y)-(PO₃H₂) (I)
wobei
X ausgewählt ist aus H, OH, Cl, F oder einer Methylgruppe
Y eine lineare Cl - C7 Alkylgruppe substituiert durch NH₂, N(CH₃)₂, NH(CH₃), N(CH₃)₃, Pyridinyl oder Imidazolyl ist,
oder pharmazeutisch verträgliche Salze oder Ester davon, enthält,
als auch wenigstens eine
amphiphile Komponente in Form eines linearen unsubstituierten C10-C20 Alkyl-Carboxylates oder Alkyl-Sulfates oder eine Mischung davon
und/oder eine wasserlösliche ionische polymere Komponente mit freien anionischen Gruppen in Form eines carboxylierten, carboxymethylierten, sulfatierten oder phosphorylierten Derivates natürlicher Polysaccharide, wobei in der Beschichtung das Amino-Bisphosphonat und die amphiphile Komponente respektive das Amino-Bisphosphonat und die wasserlösliche ionische polymere Komponente als Mischsalz mit geringer Löslichkeit in Wasser, vorliegt.

2. Implantat nach Anspruch 1, **dadurch gekennzeichnet, dass** das Mischsalz eine Löslichkeit in reinem Wasser von weniger als 1 mg/ml bei Raumtemperatur, insbesondere bevorzugt von im Bereich von weniger als 0.05 - 0.9 mg/ml bei Raumtemperatur aufweist.

3. Implantat nach einem der vorhergehenden Ansprüche, **dadurch gekennzeichnet, dass** es sich um eine glatte oder aufgeraute, poröse oder unporöse, verstärkte oder unverstärkte Trägerstruktur handelt, insbesondere ausgewählt aus der Gruppe: Gefässprothese, Knochenersatzmaterial, Knochenunterstützungsmaterial, Scaffold, Folie, Stift, Dorn, Gerüst, Nagel, Draht, Schraube, Platte, Rohr, Schlauch, Polymerschaumkomponente mit offener Porenstruktur, Faser, Gewebe, Prothesen, insbesondere Gelenkprothesen, Netze, Käfige, (cages), Metallschäume, poröse metallische und keramische Beschichtungen, allogene und xenogene Implantate, kardiovaskuläre Implantate, künstliche Bänder, künstliche Bandscheiben, intracorneale und intraokulare Implantate, cochleare Prothesen aktive Implantate, Elektroden und/oder Kombinationen davon.

4. Implantat nach einem der vorhergehenden Ansprüche, **dadurch gekennzeichnet, dass** es sich beim Amino-Bisphosphonat um Pamidronsäure, Alendronsäure, Neridronsäure, Riesedronsäure, Zoledronsäure, Olpadronsäure, Ibandronsäure, Minodronsäure oder Cimadronsäure oder eine Mischung und/oder Alkali- oder Erdalkali-Salze davon, wobei insbesondere Pamidronsäure und/oder Alendronsäure gegebenenfalls in Form des Alkali- oder Erdalkali-Salzes, bevorzugt wird.

5. Implantat nach einem der vorhergehenden Ansprüche, **dadurch gekennzeichnet, dass** das Amino-Bisphosphonat in der freien Phosphonsäure-Form, der Natrium-, Kalium-, Ammonium-, Calcium-, Magnesium- und/oder Strontium-Salzform vorliegt.

6. Implantat nach einem der vorhergehenden Ansprüche, **dadurch gekennzeichnet, dass** es sich bei der amphiphilen Komponente um Laurat, Stearat, Palmitat, Myristat, Oleat, Behenat, Dodecylsulfat bevorzugt als Alkali- oder Erdalkali-Salze oder Mischungen davon handelt.

7. Implantat nach einem der vorhergehenden Ansprüche, **dadurch gekennzeichnet, dass** es sich beim natürlichen Polysaccharid der wasserlöslichen ionischen polymeren Komponente um ein Polysaccharid ausgewählt aus Dextran, Pullulans, Chitosan, Stärke oder Cellulose oder Mischungen davon handelt.

8. Implantat nach einem der vorhergehenden Ansprüche, **dadurch gekennzeichnet, dass** das Amino-Bisphosphonat und die amphiphile Komponente in der Beschichtung in einem Molverhältnis zwischen 10 : 1 und 1 : 5 vorliegen, insbesondere bevorzugt in einem Molverhältnis von 2:1 bis 1:2.

9. Implantat nach einem der vorhergehenden Ansprüche, **dadurch gekennzeichnet, dass** das Amino-Bisphosphonat und die wasserlösliche ionische polymere Komponente in der Beschichtung in einem Molverhältnis zwischen 10 : 1 und 1 : 5, insbesondere bevorzugt in einem Molverhältnis von 2:1 bis 1:2, jeweils bezogen auf die Aminogruppen des eingesetzten Aminogruppen-haltigen Bisphosphonats und die vorhandenen anionischen Gruppen der polymeren Komponente, vorliegen.

10. Implantat nach einem der vorhergehenden Ansprüche, **dadurch gekennzeichnet, dass** die Beschichtung auf einer glatten, porösen und/oder aufgerauten Oberfläche ohne Träger oder Carrier aufgebracht ist.

11. Implantat nach einem der vorhergehenden Ansprüche, **dadurch gekennzeichnet, dass** es sich um ein Implantat auf metallischer und/oder keramischer und/oder polymerer und/oder nativer Basis handelt, wobei insbesondere bevorzugt die Beschichtung unmittelbar und ohne Zwischenschicht auf ein derartiges Implantat aufgebracht ist, und wobei es sich bevorzugtermassen beim Implantat um Calciumphosphat-Keramiken, Bioglass, Glaskeramiken, Calciumcarbonat, Calciumsulfat, organische Polymeren oder Kompositen der genannten Materialien handelt, oder um Implantatoberflächen aus Reintitan, Titanlegierungen, Kobalt-Chrom-Legierungen oder Edelstahl, oder um native Implantatoberflächen, welche bevorzugt aus Kollagen, Gelatine oder Materialien allogener Herkunft bestehen.

12. Implantat nach einem der vorhergehenden Ansprüche, **dadurch gekennzeichnet, dass** die Beschichtung nach Einsetzen in das menschliche oder tierische Gewebe respektive in den menschlichen oder tierischen Knochen das Bisphosphonat in verzögerter Weise an die Umgebung abgibt.

13. Implantat nach einem der vorhergehenden Ansprüche, **dadurch gekennzeichnet, dass** die Beschichtung eine Dicke im Bereich von 0.1 - 10, vorzugsweise von 0.5 - 5 µm aufweist.

14. Implantat nach einem der vorhergehenden Ansprüche, **dadurch gekennzeichnet, dass** es sich um eine trockene, im wesentlichen lösungsmittelfreie und im wesentlichen wasserfreie Beschichtung handelt.

15. Implantat nach einem der vorhergehenden Ansprüche, **dadurch gekennzeichnet, dass** die amphiphile Komponente anionischen Charakter aufweist, wobei sie insbesondere bevorzugt eine einwertige oder zweiwertige negative Ladung aufweist.

16. Implantat nach einem der vorhergehenden Ansprüche, **dadurch gekennzeichnet, dass** die Beschichtung als Aufschlämmung oder Suspension in einem organischen Lösungsmittel bevorzugt in einem Sprüh- oder Tauchprozess aufgetragen und anschliessend vollständig getrocknet ist.

17. Verfahren zur Herstellung eines Implantates nach einem der vorhergehenden Ansprüche, **dadurch gekennzeichnet, dass** eine Suspension oder Lösung oder ein Suspensions- oder Lösemittelgemisch hergestellt wird, welches sowohl ein Amino-Bisphosphonat der allgemeinen Formel
(H₂O₃P)-C(X)(Y)-(PO₃H₂) (I)
wobei
X ausgewählt ist aus H, OH, Cl, F oder einer Methylgruppe
Y eine lineare C1 - C7 Alkylgruppe substituiert durch NH₂, N(CH₃)₂, NH(CH₃), N(CH₃)₃, Pyridinyl oder Imidazolyl ist,
oder pharmazeutisch verträgliche Salze oder Ester davon,
als auch wenigstens eine
amphiphile Komponente in Form eines linearen unsubstituierten C10-C20 Alkyl-Carboxylates oder Alkyl-Sulfates ist oder eine Mischung davon,
und/oder eine wasserlösliche ionische polymere Komponente mit freien anionischen Gruppen in Form eines carboxylierten, carboxymethylierten, sulfatierten oder phosphorylierten Derivates natürlicher Polysaccharideenthält, und die Beschichtung durch Tauchen, Aufsprühen oder Auftropfen dieser Suspension oder Lösung oder des Suspensions- oder Lösemittelgemisches auf die zu beschichtende Oberfläche des Implantates aufgebracht wird und nach Verdampfung oder Verdunstung des Suspensions- oder Lösemittels bzw. Suspensions- oder Lösemittelgemischs eine in Wasser gering lösliche amino-bisphosphonat-haltige Beschichtung gebildet wird.

18. Verfahren nach Anspruch 17, **dadurch gekennzeichnet, dass** in einem ersten Beschichtungsschritt eine Lösung eines Amino-Bisphosphonats in einem geeigneten Lösemittel durch Tauchen, Aufsprühen oder Auftropfen auf die zu beschichtende Oberfläche aufgebracht wird und nach Verdampfung oder Verdunstung des Lösemittels in einem zweiten Beschichtungsschritt eine amphiphile und/oder polymere Komponente in einem geeigneten Lösemittel durch Tauchen, Aufsprühen oder Auftropfen auf die zuvor beschichtende Oberfläche aufgebracht wird und nach Verdampfung oder Verdunstung des zweiten Lösemittels durch in situ Salzbildung eine in Wasser gering lösliche bisphosphonat-haltige Beschichtung gebildet wird.

19. Verfahren nach einem der Ansprüche 17 oder 18, **dadurch gekennzeichnet, dass** die Konzentrationen der Beschichtungslösungen die das Amino-Bisphosphosphonat und die amphiphile und/oder die polymere Komponente enthalten, so gewählt werden, dass in der durch in situ-Salzbildung entstehenden Beschichtung das Amino-Bisphosphonat und die amphiphile Komponente in einem Molverhältnis zwischen 10 : 1 und 1 : 5, bevorzugt zwischen 2:1 und 1:2, vorliegen.

20. Verfahren nach einem der Ansprüche 17-19, **dadurch gekennzeichnet, dass** das Bisphosphonat und die amphiphile Komponente und/oder die wasserlösliche ionische polymere Komponente hergestellt werden, indem in Wasser gelöstes Bisphosphonat mit in Wasser gelöster amphiphiler Komponente respektive wasserlöslicher ionischer polymerer Komponente vermischt werden und, gegebenenfalls nach Zugabe von weiteren Salzen, wie beispielsweise Kalziumchlorid, das Fällungsprodukt als Mischsalz isoliert werden, und anschliessend dieses Mischsalz in einem Suspensions- oder Lösemittel oder Suspensions- oder Lösemittelgemisch aufgelöst respektive in diesem suspendiert werden.

21. Verfahren nach einem der Ansprüche 17-20, **dadurch gekennzeichnet, dass** als Suspensions- oder Lösemittel oder Suspensions- oder Lösemittelgemisch, Wasser oder ein oder mehrere organische Suspensions- und/oder Lösungsmittel verwendet werden, so z.B. Chloroform als Suspensionsmittel oder eine Mischung aus Chloroform und Triethylenglykol bevorzugt im Verhältnis von 97.5 : 2.5 als Lösungsmittel.

22. Verfahren nach einem der Ansprüche 17-21, **dadurch gekennzeichnet, dass** die Beschichtung als Aufschlämmung oder Suspension in einem organischen Lösungsmittel bevorzugt in einem Sprüh- oder Tauchprozess aufgetragen und anschliessend vollständig getrocknet wird.

## Claims

1. Implant, excluding a dental implant, which comprises a coating at least in surface areas which are at least in indirect contact with hard and/or soft tissue when implanted, which coating comprises an Amino-bisphosphonate of the general formula
(H₂O₃P)-C(X)(Y)-(PO₃H₂) (I)
wherein
X is selected from H, OH, Cl, F, or a methyl group
Y is a linear C1-C7 alkyl group substituted by NH₂, N(CH₃)₂, NH(CH₃), N(CH₃)₃, pyridinyl or imidazolyl,
or pharmaceutically compatible salts or esters thereof,
in addition to at least one
amphiphilic component which is a linear unsubstituted C10-C20 alkyl-carboxylate or alkyl-sulfate or a mixtures thereof,
and/or a water-soluble ionic polymeric component with free anionic groups, in the form of a carboxylated, carboxymethylated, sulphated or phosphorylated derivative of natural polysaccharides, wherein in the coating the Amino-bisphsphonate and the amphiphilic component or the Amino-bisphosphonate and the water-soluble ionic polymeric component, respectively, is present as a composite salt of low solubility in water.

2. Implant according to claim 1, **characterized in that** the composite salt, respectively, has a solubility in pure water of less than 1 mg/ml at room temperature, preferably of in the range of from less than 0.05-0.9 mg/ml at room temperature.

3. Implant according to one of the preceding claims, **characterized in that** it is a smooth or roughened, porous or non-porous, reinforced or non-reinforced support structure, preferably selected from the group: vessel prosthesis, bone replacement material, bone support material, scaffold, film, pin, thorn, rack, nail, wire, screw, plate, tube, hose, polymeric foam component with open pore structure, fibre, fabric, prostheses, especially joint prostheses, nets, cages, metal foams, porous metallic and ceramic coatings, allogenic and xenogenic implants, cardiovascular implants, artificial ligaments, artificial invertebral discs, introcorneal and intraocular implants, cochlear prostheses, active implants, electrodes and/or combinations thereof.

4. Implant according to one of the preceding claims, **characterized in that** the bisphosphonate is pamidronic acid, alendronic acid, neridronic acid, risedronic acid, zoledronic acid, olpadronic acid, ibandronic acid, minodronic acid, or cimadronic acid, or a mixture and/or alkali- or earth alkali-salts thereof, wherein especially pamidronic acid and/or alendronic acid, possibly in the form of the alkali- or earth alkali-salt is preferred.

5. Implant according to one of the preceding claims, **characterized in that** the Amino-bisphosphonate is present in the free phosphonic acid form, the sodium-, potassium-, ammonium-, calcium-, magnesium- and/or strontium-salt form.

6. Implant according to one of the preceding claims, **characterized in that** the amphiphilic component is laurate, stearate, palmitate, myristate, oleate, behenate, dodecylsulfate, preferably as alkali- or earth alkali-salts or mixtures thereof.

7. Implant according to one of the preceding claims, **characterized in that** the natural polysaccharide of the water-soluble ionic polymeric component is a polysaccharide selected from dextran, pullulans, chitosan, starch or cellulose, or mixtures thereof.

8. Implant according to one of the preceding claims, **characterized in that** the amino-bisphosphonate, and the amphiphilic componentare present in the coating in a molar ratio between 10:1 and 1:5, preferably in a molar ratio from 2:1 to 1:2.

9. Implant according to one of the preceding claims, **characterized in that** the amino-bisphosphonate and the water-soluble ionic polymeric component are present in the coating in a molar ratio between 10:1 and 1:5, preferably in a molar ratio from 2:1 to 1:2, each with respect to the amino groups of the amino group-containing bisphosphonate used and the anionic groups of the polymeric component which are present.

10. Implant according to one of the preceding claims, **characterized in that** the coating is applied to an even, porous and/or roughened surface without a support or carrier.

11. Implant according to one of the preceding claims, **characterized in that** it concerns an implant of a metallic and/or ceramic and/or polymeric and/or native basis, wherein preferably the coating is applied to such an implant directly and without an intermediate layer, and wherein the implant preferably is calcium phosphate ceramics, bioglass, glass ceramics, calcium carbonate, calcium sulphate, organic polymers, or composites of said materials, or implant surfaces of pure titanium, titanium alloys, cobalt-chromium alloys or stainless steel, or native implant surfaces, which are composed of collagen, gelatine or materials of allogenic origin.

12. Implant according to one of the preceding claims, **characterized in that** after introduction into the human or animal tissue, or the human or animal bone, respectively, the coating releases the bisphosphonate in a delayed manner into the environment.

13. Implant according to one of the preceding claims, **characterized in that** the coating has a thickness in the range of 0.1-10, preferably of 0.5-5 µm.

14. Implant according to one of the preceding claims, **characterized in that** it concerns a dry, essentially solvent-free and essentially water-free coating.

15. Implant according to one of the preceding claims, **characterized in that** the amphiphilic component has anionic character, wherein it preferably has a monovalent or bivalent negative charge.

16. Implant according to one of the preceding claims, **characterized in that** the coating is applied as a slurry or suspension in an organic solvent, preferably in a spraying- or dipping process and subsequently completely dried.

17. Method for producing an implant according to one of the preceding claims, **characterized in that** a suspension or solution or a suspension- or solvent-mixture is produced, which contains an amino-bisphosphonate of the general formula
(H₂O₃P)-C(X)(Y)-(PO₃H₂) (I)
wherein
X is selected from H, OH, Cl, F, or a methyl group
Y is a linear C1-C7 alkyl group substituted by NH₂, N(CH₃)₂, NH(CH₃), N(CH₃)₃, pyridinyl or imidazolyl,
or pharmaceutically compatible salts or esters thereof,
in addition to at least one
amphiphilic component is a linear unsubstituted C10-C20 alkyl-carboxylate or alkyl-sulfate or a mixtures thereof,
and/or a water-soluble ionic polymeric component with free anionic groups, in the form of a carboxylated, carboxymethylated, sulphated or phosphorylated derivative of natural polysaccharides,
and that the coating is applied to the implant surface to be coated by dipping, spraying, or dripping of this suspension or solution or the suspension- or solvent-mixture and that after volatilization or evaporation of the suspension or solution or the suspension- or solvent-mixture, respectively, a amino-bisphosphonate-containing coating is formed which has a low solubility in water.

18. Method according to claim 17, **characterized in that** in a first coating step a solution of an amino-bisphosphonate in a suitable solvent is applied to the surface to be coated by dipping, spraying, or dripping, and that after volatilization or evaporation of the solvent in a second coating step an amphiphilic and/or polymeric component in a suitable solvent is applied to the previously coated surface by dipping, spraying, or dripping, and that after volatilization or evaporation of the second solvent a bisphosphonate-containing coating, which has a low solubility in water, is formed by in situ salt formation.

19. Method according to one of claims 17 or 18, **characterized in that** the concentrations of the coating solutions containing the amino-bisphosphonate and the amphiphilic and/or the polymeric component are chosen such that in the coating formed by in situ-salt formation the amino-bisphosphonate and the amphiphilic, component are present in a molar ratio between 10:1 and 1:5, preferably between 2:1 and 1:2.

20. Method according to one of claims 17-19, **characterized in that** the bisphosphonate and the amphiphilic component and/or the water-soluble ionic polymeric component are produced by mixing bisphosphonate solubilized in water with amphiphilic component solubilized in water or water-soluble ionic polymeric component, respectively, and, possibly after the addition of additional salts, as for example calcium chloride, the precipitation product is isolated as a composite salt, and subsequently this composite salt is solubilized in a suspension means or solvent or a suspension- or solvent mixture or suspended therein, respectively.

21. Method according to one of claims 17-20, **characterized in that** water or one or more organic suspension means and/or solvents are used as suspension means or solvents or suspension- or solvent mixtures, such as e.g. chloroform as a suspension means or a mixture of chloroform and triethyleneglycol preferably in the ratio of 97.5:2.5 as a solvent.

22. Method according to one of claims 17-21, **characterized in that** the coating is applied as a slurry or suspension in an organic solvent, preferably in a spraying- or dipping process and subsequently completely dried.

## Revendications

1. Implant, sauf implant dentaire, présentant au moins dans certaines' zones des parties de sa surface qui se trouvent au moins indirectement en contact avec des tissus durs et/ou mous, un revêtement lequel contient à la fois un amino-bis-phosphonate répondant à la formule générale
(H₂O₃P)-C(X)(Y)-(PO₃H₂) (I)
dans laquelle
X est choisi parmi H, OH, Cl, F ou un groupe méthyle
y est un groupe alkyle linéaire en C1 à C7, substitué avec NH₂, N(CH₃)₂, NH(CH₃), N(CH₃)₃, pyridinyle ou imidazolyle,
ou ses sels ou esters pharmaceutiquement acceptables,
et au moins un
composant amphiphile sous forme d'un carboxylate d'alkyle en C10 à C20 ou sulfate d'alkyle linéaire non-substitué ou d'un de leurs mélanges
et/ou un composant polymère ionique hydrosoluble pourvu de groupes anioniques libres, sous forme d'un dérivé carboxylé, carboxyméthylé, sulfaté ou phosphorylé de polysaccharides naturels, l'amino-bis-phosphonate et le composant amphiphile ou bien l'amino-bis-phosphonate et le composant polymère ionique hydrosoluble se présentant au sein dudit revêtement sous forme d'un sel mixte ayant une faible solubilité dans l'eau.

2. Implant selon la revendication 1, **caractérisé en ce que** ledit sel mixte présente dans de l'eau pure à température ambiante une solubilité qui est inférieure à 1 mg/ml et qui est de préférentiellement comprise entre moins de 0,05 et 0,9 mg/ml à température ambiante.

3. Implant selon l'une des revendications précédentes, **caractérisé en ce qu'**il s'agit d'une structure de support de nature lisse ou rugueuse, poreuse ou non-poreuse, renforcée ou non-renforcée, choisie notamment dans le groupe constitué par les éléments suivants : prothèse vasculaire, matériau de substitution osseuse, matériau de support osseux, échafaudage de tissu, feuille, goupille, poinçon, squelette, clou, fil métallique, vis, plaque, tube, tuyau, composant en mousse polymère à pores ouverts, fibre, tissu, prothèses, notamment prothèses d'articulation, filets, cages, mousses métalliques, revêtements poreux métalliques et céramiques, implants allogènes et xénogènes, implants cardiovasculaires, ligaments artificiels, disques intervertébraux artificiels, implants intracornéens et intraoculaires, prothèses cochléaires, implants actifs, électrodes et/ou leurs combinaisons.

4. Implant selon l'une des revendications précédentes, **caractérisé en ce que** l'amino-bis-phosphonate se présente sous forme d'acide pamidronique, d'acide alendronique, d'acide néridronique, d'acide risédronique, d'acide zolédronique, d'acide olpadronique, d'acide ibandronique, d'acide minodronique ou d'acide cimadronique ou d'un de leurs mélanges et/ou sels alcalins ou alcalinoterreux, s'agissant avec une préférence particulière d'acide pamidronique et/ou d'acide alendronique, le cas échéant sous forme de leur sel alcalin ou alcalinoterreux.

5. Implant selon l'une des revendications précédentes, **caractérisé en ce que** l'amino-bis-phosphonate se présente sous forme de l'acide phosphoreux libre, sous forme d'un sel de sodium, de potassium, d'ammonium, de calcium, de magnésium et/ou de strontium.

6. Implant selon l'une des revendications précédentes, **caractérisé en ce que** le composant amphiphile est un laurate, stéarate, palmitate, myristate, oléate, béhénate, dodécylsulfate, de préférence sous forme de sels alcalins ou alcalinoterreux, ou un de leurs mélanges.

7. Implant selon l'une des revendications précédentes, **caractérisé en ce que** le polysaccharide naturel du composant polymère ionique hydrosoluble est un polysaccharide choisi parmi le dextrane, le pullulane, le chitosane, l'amidon ou le cellulose ou l'un de leurs mélanges.

8. Implant selon l'une des revendications précédentes, **caractérisé en ce que** ledit revêtement contient l'amino-bis-phosphonate et le composant amphiphile selon un rapport molaire entre 10 : 1 et 1 : 5, avec une préférence particulière selon un rapport molaire compris entre 2 : 1 et 1 : 2.

9. Implant selon l'une des revendications précédentes, **caractérisé en ce que** ledit revêtement contient l'amino-bis-phosphonate et le composant polymère ionique hydrosoluble selon un rapport molaire entre 10 : 1 et 1 : 5, avec une préférence particulière selon un rapport molaire compris entre 2 : 1 et 1 : 2, exprimé respectivement par rapport aux groupes amino du bis-phosphonate contenant des groupes amino et aux groupes anioniques présents sur le composant polymère.

10. Implant selon l'une des revendications précédentes, **caractérisé en ce que** ledit revêtement est appliqué sur une surface lisse, poreuse et/ou rugueuse, sans support ni élément de portage.

11. Implant selon l'une des revendications précédentes, **caractérisé en ce qu'**il s'agit d'un implant ayant une base métallique et/ou céramique et/ou polymère et/ou native, ledit revêtement étant avec une préférence particulière appliqué directement et sans couche intermédiaire sur un tel implant, et ledit implant étant préférentiellement une céramique en phosphate de calcium, un verre bioactif, une vitrocéramique, du carbonate de calcium, du sulfate de calcium, un polymère organique ou un matériau composite à base des matériaux précités, ou qu'il s'agit d'une surface d'implant réalisée en titane pur, en alliages de titane, en alliages de cobalt-chrome ou en acier inoxydable, ou de surfaces d'implant natives réalisées de préférence en collagène, gélatine, ou matériaux d'origine allogène.

12. Implant selon l'une des revendications précédentes, **caractérisé en ce que**, suite à l'implantation dans le tissu humain ou animal ou bien dans l'os humain ou animal, ledit revêtement libère d'une manière retardée le bis-phosphonate dans l'environnement.

13. Implant selon l'une des revendications précédentes, **caractérisé en ce que** le revêtement présente une épaisseur comprise entre 0,1 et 10, de préférence entre 0,5 et 5 µm.

14. Implant selon l'une des revendications précédentes, **caractérisé en ce qu'**il s'agit d'un revêtement sec, sensiblement exempt de solvants et sensiblement anhydre.

15. Implant selon l'une des revendications précédentes, **caractérisé en ce que** le composant amphiphile revet un caractère anionique, présentant avec une préférence particulière une simple ou double charge négative.

16. Implant selon l'une des revendications précédentes, **caractérisé en ce que** ledit revêtement est appliqué sous forme d'une bouillie ou d'une suspension à base d'un solvant organique, de préférence selon un processus d'atomisation ou d'immersion, pour ensuite être complètement séché.

17. Procédé de fabrication d'un implant selon l'une des revendications précédentes, **caractérisé en ce que** l'on prépare une suspension ou solution ou un mélange d'agents de suspension ou de solution contenant à la fois un amino-bis-phosphonate répondant à la formule générale
(H₂O₃P)-C(X)(Y)-(PO₃H₂) (I)
dans laquelle
X est choisi parmi H, OH, Cl, F ou un groupe méthyle
y est un groupe alkyle linéaire en C1 à C7, substitué avec NH₂, N(CH₃)₂, NH(CH₃), N(CH₃)₃, pyridinyle ou imidazolyle,
ou ses sels ou esters pharmaceutiquement acceptables,
et au moins un
composant amphiphile sous forme d'un carboxylate d'alkyle en C10 à C20 ou sulfate d'alkyle linéaire non-substitué ou d'un de leurs mélanges,
et/ou un composant polymère ionique hydrosoluble pourvu de groupes anioniques libres, sous forme d'un dérivé carboxylé, carboxyméthylé, sulfaté ou phosphorylé de polysaccharides naturels, et que l'on applique ledit revêtement sur la surface à revêtir de l'implant en y disposant cette suspension ou solution ou ce mélange d'agents de suspension ou de solution par immersion, atomisation ou dépôt de gouttes pour ainsi former, suite à l'évaporation naturelle ou accélérée de l'agent de suspension ou de solution et/ou du mélange d'agents de suspension ou de solution, un revêtement contenant de l'amino-bis-phosphonate et présentant une faible solubilité dans l'eau.

18. Procédé selon la revendication 17, **caractérisé en ce que**, dans une-première étape de revêtement, on applique une solution d'un amino-bis-phosphonate dans un solvant approprié sur la surface à revêtir en y disposant celleci-ci par immersion, atomisation ou dépôt de gouttes et que, dans une deuxième étape mise en oeuvre suite à la l'évaporation naturelle ou accélérée du solvant, on applique un composant amphiphile et/ou polymère dans un solvant approprié la surface précédemment revêtue en y disposant celui-ci par immersion, atomisation ou dépôt de gouttes et que, suite à la l'évaporation naturelle ou accélérée du deuxième solvant, on obtient, par formation de sel in-situ, un revêtement contenant de l'amino-bis-phosphonate et présentant une faible solubilité dans l'eau.

19. Procédé selon l'une des revendications 17 ou 18, **caractérisé en ce que** les concentrations des solutions de revêtement, lesquelles contiennent l'amino-bis-phosphonate et le composant amphiphile et/ou polymère, sont choisies telles que le revêtement obtenu par formation de sel in-situ contient l'amino-bis-phosphonate et le composant amphiphile selon un rapport molaire entre 10 : 1 et 1 : 5, de préférence entre 2 : 1 et 1 : 2.

20. Procédé selon l'une des revendications 17 à 19, **caractérisé en ce que** le bis-phosphonate et le composant amphiphile et/ou le composant polymère ionique hydrosoluble sont préparés en mélangeant le bis-phosphonate dissous dans l'eau avec le composant amphiphile ou le composant polymère ionique hydrosoluble dissous dans l'eau puis en isolant, le cas échéant suite à l'ajout d'autres sels comme par exemple le chlorure de calcium, le précipité sous forme d'un sel mixte pour ensuite dissoudre ou mettre en suspension ledit sel mixte dans un agent de suspension ou de solution ou dans un mélange d'agents de suspension ou de solution.

21. Procédé selon l'une des revendications 17 à 20, **caractérisé en ce que** l'on utilise, en tant qu'agent de suspension ou de solution ou mélange d'agents de suspension ou de solution, de l'eau ou bien un ou plusieurs agents de suspension ou de solution organiques, comme par exemple le chloroforme en tant qu'agent de suspension ou un mélange de chloroforme et de triéthylèneglycol en tant que solvant, le rapport de ce dernier étant préférentiellement de 97,5 : 2,5.

22. Procédé selon l'une des revendications 17 à 21, **caractérisé en ce que** ledit revêtement est appliqué sous forme d'une bouillie ou d'une suspension à base d'un solvant organique, de préférence selon un processus d'atomisation ou d'immersion, pour ensuite être complètement séché.
